# EUROPEAN PATENT APPLICATION

(11) **EP 4 696 956 A1**
(43) Date of publication of application: **18.02.2026**
(21) Application number: 25196056.3
(22) Date of filing: 14.08.2025
(51) Int. Cl.: F25B 49/00, F24F 11/36, F25B 49/02, F25B 49/04, H02J 9/06, H02J 7/00

(54) **A2L GAS SENSOR SERVICE BACKUP SAFETY**

(30) Priority: 15.08.2024 US 202463683360 P; 06.08.2025 US 202519292621
(71) Applicant: Vertiv Corporation, Westerville, OH 43082 (US)
(72) Inventor: KING, Jeremy, Westerville, 43082 (US); RAVEN, Matthew A., Westerville, 43082 (US); BLASER, Colin L., Westerville, 43082 (US)
(74) Representative: Beal, James Michael

(57) **Abstract**

A system may include a sensor communicatively coupled to a cooling system controller and configured to detect a refrigerant. The system may include an alarm coupled to the sensor and configured to receive an alarm signal from the sensor when the refrigerant is detected. The system may include a battery configured to power the sensor and the alarm with secondary power if primary power is disrupted. The system may include a sensor controller coupled to the sensor, the alarm, and the cooling system controller, and configured to receive primary power and distribute the primary power to the sensor and the alarm, the sensor controller configured to execute one or more steps comprising: detecting a disruption or a planned disruption of the primary power; and upon a detection of the disruption, or the planned disruption, of the primary power, causing the sensor controller to receive the secondary power from the battery.

## Description

### TECHNICAL FIELD

The present disclosure relates to gas sensor/alarm systems and more particularly to backup power systems for gas sensor/alarm systems.

### BACKGROUND

Cooling systems, such as cooling systems for data centers, often require the use of refrigerants. The refrigerants operate as a working fluid within the refrigerant cycle of cooling systems, undergoing repeated phase transitions between liquid and gas.

Due to possible toxicity, flammability, and environmental damage from refrigerants, cooling systems often use gas sensors and alarms to determine whether the refrigerant is leaking from the cooling system. While keeping these sensors and alarms online and powered is important for safety, sensors and alarms for the cooling system are often taken offline during maintenance of the cooling system. The offline status of the sensor and alarms during maintenance is a particular concern, as there is both an increased risk of a leak of refrigerant from a cooling system during a maintenance event and an increased risk of a technician being in the vicinity of the cooling system when the leak occurs during the maintenance event. Therefore, there is a need for a system and method that ensures that a gas sensor and alarm of a cooling system are powered while the cooling system is taken offline.

### SUMMARY

In some aspects, the techniques described herein relate to a system including: a sensor subsystem configured to be selectively powered by primary power and secondary power including: a sensor communicatively coupled to a cooling system controller and configured to detect a refrigerant; an alarm coupled to the sensor and configured to receive an alarm signal from the sensor when the refrigerant is detected; a battery configured to power the sensor and the alarm with secondary power if primary power is disrupted; and a sensor controller communicatively coupled to the sensor, the alarm, and the cooling system controller, and configured to receive primary power and distribute the primary power to the sensor and the alarm, the sensor controller configured to execute one or more steps including: at least one of detecting a disruption of the primary power or determining a planned disruption of the primary power; and upon a detection of the disruption of the primary power or a determination of the planned disruption, of the primary power, causing the sensor controller to receive the secondary power from the battery.

In some aspects, the techniques described herein relate to a system including: a cooling system including: a refrigerant; and a cooling system controller; and a sensor subsystem including: a sensor communicatively coupled to the cooling system controller and configured to detect the refrigerant; an alarm coupled to the sensor and configured to receive an alarm signal from the sensor when the refrigerant is detected; a battery configured to power the sensor and the alarm with secondary power if primary power is disrupted; and a sensor controller communicatively coupled to the sensor, the alarm, and the cooling system controller, and configured to receive primary power and distribute the primary power to the sensor and the alarm, the sensor controller including one or more processors, whereupon a disruption, or a planned disruption, of power to the cooling system, the one or more processors are configured to execute a set of program instructions stored in memory, the set of program instructions configured to cause the one or more processors to: at least one of detect the disruption or the primary power or determine the planned disruption, of the primary power; and upon a detection of the disruption of the primary power or a determination of the planned disruption of the primary power, cause the sensor controller to receive the secondary power from the battery.

In some aspects, the techniques described herein relate to a method of switching a power source for a refrigerant sensor in a cooling system including: operating a system controller and a sensor controller with primary power; at least one of detecting a disruption of the primary power or determining a planned disruption, of the primary power by the sensor controller; and upon the disruption, or the planned disruption, of the primary power, causing the sensor controller to receive secondary power from a battery.

It is to be understood that both the foregoing general description and the following detailed description are exemplary and explanatory only and are not necessarily restrictive of the present disclosure. The accompanying drawings, which are incorporated in and constitute a part of the specification, illustrate subject matter of the disclosure. Together, the descriptions and the drawings serve to explain the principles of the disclosure.

### BRIEF DESCRIPTION OF THE DRAWINGS

The numerous advantages of the disclosure may be better understood by those skilled in the art by reference to the accompanying figures.

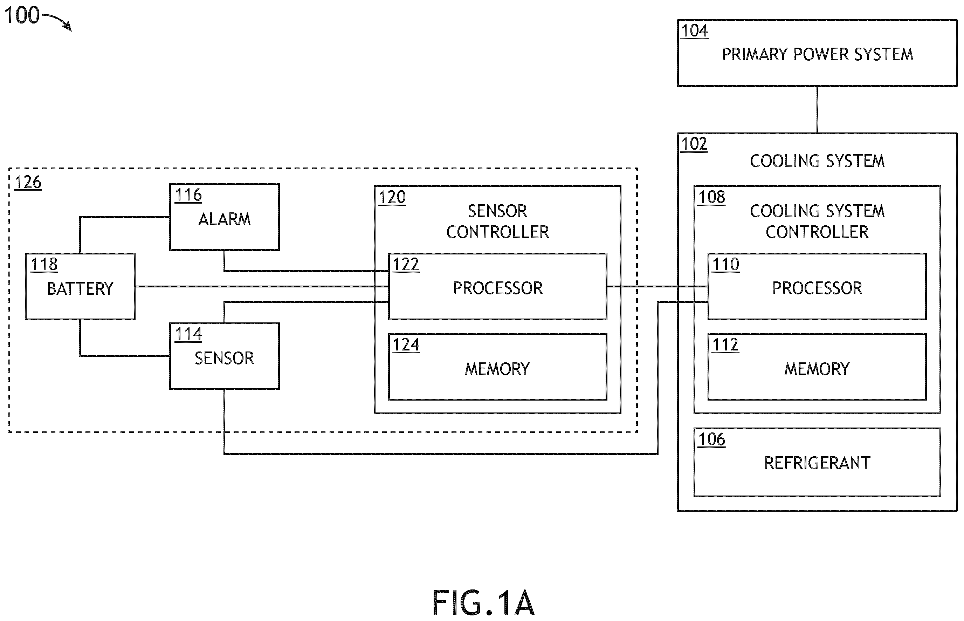

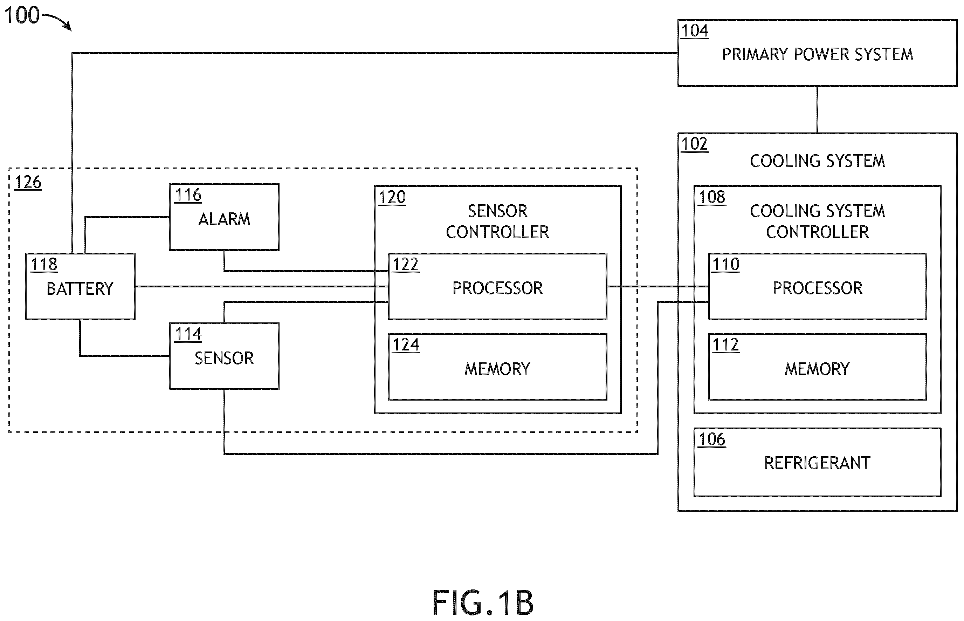

It is further noted that, while FIG. 1 depicts the sensor controller as being embodied separately from the cooling system controller, such a configuration of system is not a limitation on the scope of the present disclosure, but is provided merely for illustrative purposes.
FIG. 1A illustrates a conceptual view of a system for providing backup power and control for gas refrigerant sensing in a cooling system, in accordance with one or more embodiments of the disclosure.
FIG. 1B illustrates a conceptual view of the system with the battery electrically coupled to the primary power system, in accordance with one or more embodiments of the disclosure.
FIG. 2 illustrates a perspective view of a cooling system, in accordance with one or more embodiments of the disclosure.
FIG. 3 illustrates a process flow diagram depicting a method for switching a power source for a sensor from primary power to secondary power, in accordance with one or more embodiments of the disclosure.

### DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein, a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by any one of the following: A is true (or present), and B is false (or not present), A is false (or not present), and B is true (or present), and both A and B are true (or present).

In addition, the use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein, any reference to "one embodiment" or "embodiments" means that a particular element, feature, structure, or characteristic described in connection with the embodiment is included in at least one embodiment disclosed herein. The appearances of the phrase "in embodiments" in various places in the specification are not necessarily all referring to the same embodiment, and embodiments may include one or more of the features expressly described or inherently present herein, or any combination or sub-combination of two or more such features, along with any other features which may not necessarily be expressly described or inherently present in the instant disclosure.

Reference will now be made in detail to the subject matter disclosed, which is illustrated in the accompanying drawings.

FIGS. 1A through 3 generally illustrate a system and method for providing backup power to a refrigerant gas sensor and associated alarm, in accordance with one or more embodiments of the disclosure. The system includes a sensor controller communicatively coupled to a refrigerant gas sensor, an alarm that activates when refrigerant gas is detected, a backup battery, and a cooling system controller. The sensor controller is configured to detect if the cooling system is online (e.g., receiving primary power). If the sensor controller detects that primary power to the sensor controller is disrupted, the sensor controller will cause the refrigerant gas sensor and alarm to rely on secondary power supplied by the backup battery.

The embodiments of the present disclosure are particularly advantageous, as the system sustains power to the refrigerant gas sensor and alarm while primary power to the cooling system is disrupted. For example, when replacing a failed component of the cooling system, such as a valve, or compressor, and the primary power to the cooling system is turned off, the system will still be able to sense a refrigerant gas leak, reducing toxicity and/or fire risks.

FIG. 1A illustrates a conceptual view of a system 100 for providing backup power and control for gas refrigerant sensing in a cooling system 102, in accordance with one or more embodiments of the disclosure. The cooling system 102 may be configured for use in any type of cooling scheme. For example, the cooling system 102 may be configured to cool electronic equipment, such as electronic equipment in a data center. The cooling system 102 may be powered by a primary power system 104. For example, the cooling system 102 may be powered by utility power or by power that has been converted from utility power.

In embodiments, the cooling system 102 includes refrigerant 106 used in the refrigeration cycle. When in use, the refrigerant 106 may undergo phase transitions between a liquid and gas, with the gas phase of the refrigerant 106 often having a risk of leaking from the cooling system 102. The refrigerant 106 may include any type of refrigerant 106 used for cooling. For example, the refrigerant 106 may include refrigerants classified by the American Society of Heating Refrigerating and Air-Conditioning Engineers (ASHRAE) including, but not limited to, Group A refrigerants (e.g., single-component refrigerants, such as hydrocarbons like propane (R-290) and isobutane (R-600a)), Group B refrigerants (e.g., azeotropic blends, having a constant boiling point and composition at a given pressure, which include R-500 series refrigerants like R-502), Group C refrigerants (e.g., zeotropic blends having varying compositions and boiling points at a given pressure, such as R-400 series refrigerants like R-404A and R-407C, Group D refrigerants(e.g., refrigerants with glide values greater than 0.5 K at saturation conditions, such as R-407A, Group E refrigerants (e.g., refrigerants with glide values less than or equal to 0.5 K at saturation conditions, such as R-407C), and Group R refrigerants (e.g., reclaimed refrigerants).

In embodiments, the refrigerant 106 includes A2L refrigerants, as classified by the American Society of Heating, Refrigerating and Air-Conditioning Engineers (ASHRAE). For example, the refrigerant 106 may include, but not be limited to, R-32 (difluoromethane), R-1234yf (2,3,3,3-tetrafluoropropene), R-1234ze (trans-1,3,3,3-tetrafluoropropene), R-454B (azeotropic blend), R-455A (azeotropic blend), and R-457A (azeotropic blend). A2L refrigerants are a category of mildly flammable refrigerants that pose a reduced risk of ignition compared to highly flammable refrigerants, but still require precautions due to their flammability.

In embodiments, the cooling system 102 includes a cooling system controller 108. The cooling system controller 108 includes one or more processors 110 and memory 112. For example, the memory 112 may maintain program instructions configured to cause the one or more processors 110 to carry out any of the one or more process steps (e.g., controlling the refrigeration cycle of the cooling system) or other process steps as described throughout the present disclosure. The cooling system controller 108 may include or be part of an electronic thermal management system capable of controlling multiple cooling units, such as the iCOM^{™} thermal system control manufactured by the Vertiv company.

In embodiments, the system 100 includes one or more sensors 114 for detecting refrigerant 106, such as refrigerant 106 in the gas phase. The one or more sensors 114 may be configured to detect any type of refrigerant 106, such as one or more types of refrigerants 106 as described herein. The sensor 114 may include any gas sensor type including, but not limited to, photoionization (PID) sensors, semiconductor sensors, electrochemical sensors, ultrasonic sensors, and infrared sensors. Upon detecting the refrigerant 106, the one or more sensors 114 are configured to transmit a sensor signal that is sent to either the sensor controller 120, the cooling system controller 108, or one or more alarms 116 (e.g., the sensor signal received by the one or more alarms 116 as an alarm signal).

In embodiments, the system 100 includes one or more alarms 116 communicatively coupled to the one or more sensors 114. For example, the one or more alarms may be configured to broadcast an alert to one or more technicians if leaked refrigerant 0106 is detected by the one or more sensors 114. The one or more alarms 116 may include one or more aural alarms, one or more visual alarms, and/or one or more haptic alarms. For example, the one or more alarms 116 may include an audible alarm that broadcasts an alert noise. In another example, the one or more alarms 116 may include a warning light. In another example, the alarm is an image or text that is displayed on a display. In another example, the one or more alarms 116 emits both an audible alarm and a visual alarm.

In embodiments, the system 100 includes a battery 118 configured to provide secondary power, or backup power, to one or more components of the system 100 (e.g., the one or more sensors 114 and one or more alarms 116 if primary power to the cooling system 102 is interrupted. The battery 118 may be of any battery type, including but not limited to, lithium-ion batteries, lead-acid batteries, nickel-cadmium batteries, nickel-hydride batteries, lithium polymer batteries, and lithium iron phosphate batteries.

In embodiments, the system 100 includes a sensor controller 120 communicatively coupled to the sensor controller 120, the sensor 114, and the alarm 116. The sensor controller 120 is configured to receive power (e.g., DC power) from the battery 118 when the primary power to the cooling system is disrupted. In embodiments, the sensor controller 120 is communicatively coupled to the battery 118. For example, the sensor controller 120 may transmit a signal to the battery that primary power has been disrupted, causing the battery 118 to transmit power to the sensor controller 120. In embodiments, the battery 118 is charged via power transmitted from the sensor controller 120 when primary power is not disrupted. The sensor controller 120 includes one or more processors 122 and memory 124. For example, the memory 112 may maintain program instructions configured to cause the one or more processors 122 to carry out any of the one or more process steps as described throughout the present disclosure. The sensor controller 120 may be configured as a control board that includes gas sensor detection logic and communication protocol. In alternative embodiments, the sensor controller 120 includes electronic circuitry for power detection and power switching without memory 112.

The sensor controller 120 is communicatively coupled to one or more components of the system 100 via various wired modes. For example, the sensor controller 120 may be communicatively coupled to one or more of the sensors 114, the alarms 116, the battery 118, or the cooling system controller 108 via a wireline connection using a client/server communications protocol, such as ModBUS. For instance, the system 100 may include a sensor controller 120 that is communicatively coupled to the cooling system controller 108 and the one or more sensors 114 via ModBUS. In another example, the sensor controller 120 may be communicatively coupled to one or more of the one or more sensors 114, the one or more alarms 116, the battery 118, or the cooling system controller 108 via a digital signal. For instance, the sensor controller 120 may be communicatively coupled to the one or more alarms 116 via a digital signal.

It should be understood that the system 100 may include one or more of the one or more sensors 114, the one or more alarms 116, the battery 118, the sensor controller 120, the cooling system controller 108, and the cooling system 102. For example, the system 100 may include a sensor subsystem 126 that includes one or more of the one or more sensors 114, the one or more alarms 116, the battery 118, and the sensor controller 120. The sensor subsystem 126 may be configured to be selectively powered by primary power and secondary power. It should also be understood that any of the components of the system 100 may receive secondary power, or transmit secondary power from, any of the other components of the system 100. For example, the one or more sensors 114 and the one or more alarms 116 may be configured to receive power directly from the battery 118. In another example, the sensor controller 120 receives secondary power from the battery 118, and transmits and/or distributes the secondary power to the one or more sensors 114 and/or the one or more alarms 116. It should also be understood that any of the components of the system 100 may be communicatively coupled to any of the other components of the system 100. For example, one or more of the one or more sensors 114 may be communicatively coupled to both the cooling system controller 108 and the sensor controller 120. The sensor subsystem 126 may be integrated within, or implemented adjacent to, the cooling system 102.

FIG. 1B illustrates a conceptual view of the system 100 with the battery 118 electrically coupled to the primary power system 104, in accordance with one or more embodiments of the disclosure. For example, during normal operation, primary power from the primary power system 104 may flow to the system controller 108 as well as to the battery 118, maintaining the charge of the battery 118 until a disruption occurs. During a disruption, such as when a component from the cooling system 102 needs servicing and the electrical connection between the primary power system 104 and the cooling system needs to be temporarily disconnected, the battery 118 may then supply power to the sensor 114, the alarm, and/or the sensor controller 120 to continuously monitor for refrigerant leaks and notify a user of the leak. The battery 118 may be electrically coupled to other system components including, but not limited to, the cooling system controller 108. For example, the cooling system controller 108 may control aspects of the charging and/or discharging of the battery 118.

In embodiments, charging the battery 118 via the primary power system 104 includes converting the primary power to a power capable of charging the battery 118. For example, if the primary power includes alternating current (AC), the primary power may be converted to direct current (DC) via one or more inverters. In another example, a DC power received from the primary power system 104 may be converted to a more battery-compatible DC power via a DC/DC converter.

In embodiments, the one or more processors 122 of the sensor controller 120 are configured to detect a disruption of primary power or determine a planned disruption (e.g., a scheduled disruption) of primary power. For example, the sensor controller 120 may detect a loss of signal from the cooling system controller 108 (e.g., via ModBUS), indicating a disruption of power by the cooling system 102. In another example, the sensor controller 120 may receive a "notice to disrupt power" signal from the cooling system controller 108 followed by a disruption of power, indicating a planned disruption of power by the cooling system 102. In another example, the one or more processors 122 of the sensor controller 120 may include circuitry that continually monitors the status of primary power within the system 100. For instance, one or more processors 122 may be communicatively coupled to one or more electrical sensors that can detect changes in electrical parameters such as voltage, frequency, amplitude, and/or phase angle. The electrical sensors may provide real-time feedback, enabling the sensor controller 120 to infer a decision regarding detection of a disruption, or a determination of a planned disruption, and switching from primary power to secondary power.

As used herein, the term "planned disruption" refers to any disruption in the primary power that has been scheduled ahead of time. For example, when the system 100 determines, via the one or more processors 110,122, that a planned disruption is scheduled to occur, the one or more processors 110, 122 will coordinate the shutting off of primary power with the powering of the sensor controller 120 by the battery 118 so that there is no loss of power to the sensor subsystem 126 will during the change from primary power to secondary power.

In embodiments, the one or more processors 122 of the sensor controller 120 are configured to, upon a detection of a disruption or primary power or a determined planned disruption of primary power, cause the sensor controller to receive secondary power from the battery 118. Once the secondary power is received by the sensor controller 120, the sensor controller 120 can keep in communication with the one or more sensors 114 and/or the one or more alarms 116 and may also direct secondary power to the one or more sensors 114 and/or one or more alarms 116. For example, the one or more processors 122 may be communicatively coupled to one or more switching components, such as solid-state relays (SSRs) that can be employed to seamlessly transfer the load from the primary source to the battery 118.

In embodiments, the one or more processors 122 of the controller 120 are configured to stop the flow of secondary power from the battery 118 once primary power is detected. For example, after maintenance has been performed on a powered-down cooling system 102, primary power may again be used to power the cooling system 102. The sensor controller 120 may then receive a signal from the cooling system controller 108 indicating that primary power has resumed. The sensor controller 120 may then, via the one or more processors 122, cause the sensor controller 120 to no longer receive secondary power from the battery 118, and cause the one or more sensors 114 and the one or more alarms 116 to be powered by primary power. The primary power received by the one or more sensors 114, the one or more alarms 116 may be received from the cooling system controller 108, the sensor controller 120, or by some other circuitry within the cooling system 102.

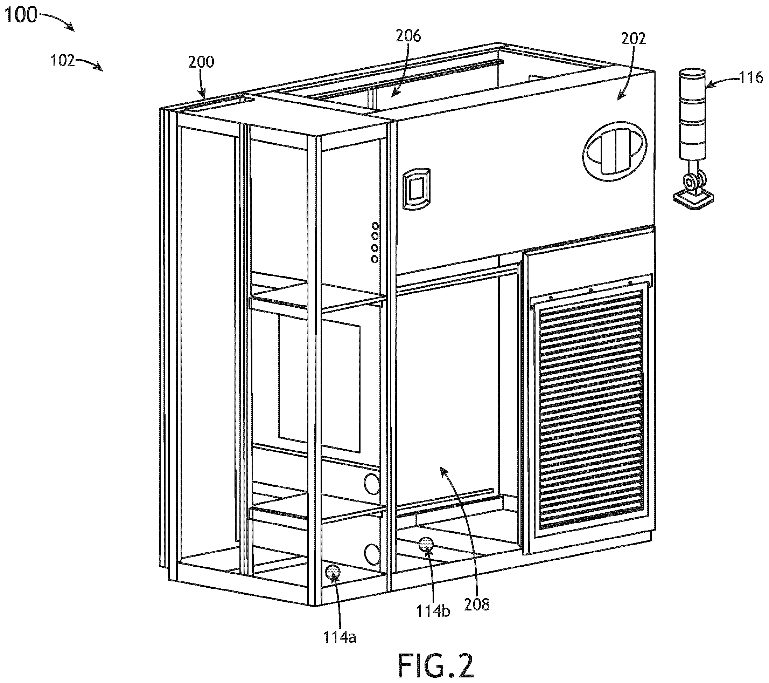

FIG. 2 illustrates a perspective view of a cooling system 102, in accordance with one or more embodiments of the disclosure. The cooling system 102 may include or be integrated within the system 100 as described herein. The cooling system 102 may be used to cool electronic equipment, such as servers in a data center. The cooling system 102 may include a cabinet 200 that stores one or more components of the system 100. For example, the cabinet 200 may include an electric panel 202 that stores the cooling system controller 108, battery 118, and/or sensor controller 120. In another example, the cabinet 200 may include one or more sensors 114a-b. The system 100 may also include one or more alarms 116 within or adjacent to the cabinet 200. The cabinet 200 may also include a compression section 204 where the refrigerant 106 is compressed during the compression cycle, a fan section 206, and an airflow section 208 that allows the flow of air from one or more fans of the fan section 206.

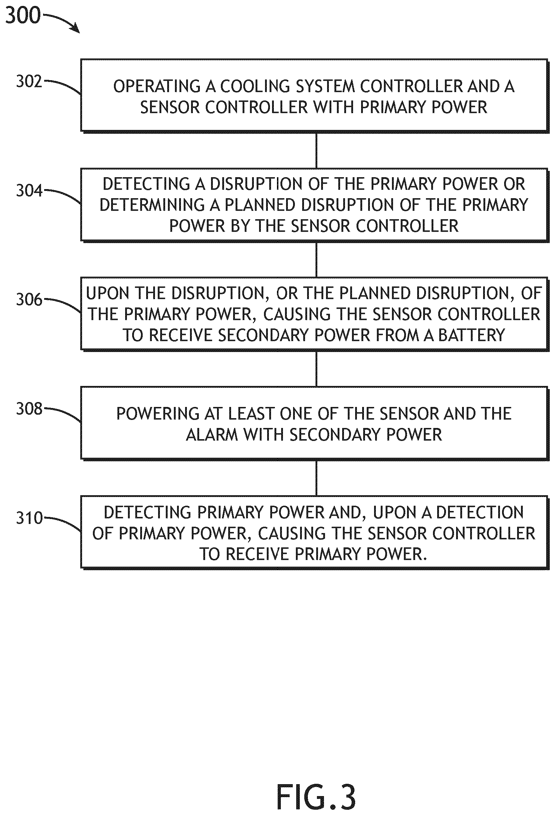

FIG. 3 illustrates a process flow diagram depicting a method 300 for switching a power source for a sensor 114 (e.g., a refrigerant sensor) from primary power to secondary power, in accordance with one or more embodiments of the disclosure. For example, the method 300 may be used switch the power of the one or more sensors 114 while the cooling system 102 is in the process of being disconnected from the primary power system 104, such as when the cooling system 102 is undergoing maintenance. The method 300 may also switch the power source of other components of the system, including, but not limited to, the alarm 116 and sensor controller 120.

In embodiments, the method 300 includes a step 302 of operating a cooling system controller 108 and a sensor controller 120 with primary power. For example, the cooling system controller 108 may receive primary power via the primary power system 104 and the sensor controller 120 may receive primary power from the cooling system 102, such as via the cooling system controller 108.

In embodiments, the method 300 includes a step 304 of detecting a disruption of primary power or determining a planned disruption of primary power by the sensor controller 120. For example, the sensor controller 120 may detect a disruption, or a planned disruption, of primary power and/or signal communication from the cooling system controller 108. In another example, the sensor controller 120 may receive a signal communication from the cooling system controller 108 signaling that primary power will be disrupted. For instance, control circuitry in the sensor controller 120 may continuously monitor the status of primary power in the cooling system 102 and use algorithms to determine whether primary power has been or will disrupted. The control circuitry may also be used to determine timing for transitioning between the primary power and the secondary power.

In embodiments, the method 300 includes a step 306 of, upon a disruption, or a planned disruption, of primary power, causing the sensor controller 120 to receive secondary power from a battery 118. For example, the sensor controller 120 may include switching devices such as SSRs to transfer the load from primary power to secondary power for the sensor controller 120 based on data gained from the detection step 304.

In embodiments, the method 300 includes a step 308 of powering at least one of the sensor 114 and the alarm 116 with secondary power (e.g., receiving secondary power from the sensor controller 120). For example, when primary power is disrupted, the one or more sensors 114 and/or the one or more alarms 116 may switch to receiving secondary power from the battery 118 or receiving secondary power from the sensor controller 120 (e.g., the sensor controller 120 having received secondary power from the battery 118). The switch from using primary power to secondary power for the one or more sensors 114 and/or the one or more alarms 116 may be controlled via switching power as described herein. For example, the switching devices within the sensor controller 120 may be used to switch power for the one or more sensors 114 and/or the one or more alarms 116 from primary power to secondary power. In another example, the one or more sensors 114 and/or the one or more alarms 116 may include switching devices that enable the one or more sensors 114 and/or the one or more alarms 116 to switch from primary power to secondary power.

In embodiments, the method 300 includes a step of detecting a refrigerant by the refrigerant sensor 116, wherein the refrigerant comprises an A2L refrigerant. In embodiments, the method 300 includes a step of activating an alarm 116 based on a detection of the A2L refrigerant by the refrigerant sensor.

In embodiments, the method 300 includes a step 310 of detecting primary power and, upon a detection of primary power, causing the sensor controller 120 to receive primary power. For instance, the controller circuitry in the sensor controller 120 may be configured to detect a resumption of primary power. Switching devices in the sensor controller 120 may then be used to switch the power for the sensor controller 120 from secondary power to primary power. Additionally, the one or more sensors 114 and/or one or more alarms 116 may switch from using secondary power to primary power based on the control circuitry and switching devices of the sensor controller 120 and/or control circuitry and/or switching devices within the one or more sensors 114 and/or one or more alarms 116.

While implementations of the method 300 are discussed herein, it is further contemplated that various steps of the method 300 may be included, excluded, rearranged, and/or implemented in many ways without departing from the essence of the present disclosure. Accordingly, the foregoing embodiments and implementations of the method 300 are included by way of example only and are not intended to limit the present disclosure in any way.

The one or more processors 110, 122 may include any one or more processing elements known in the art. In this sense, the one or more processors 110, 122 may include any microprocessor-type device configured to execute software algorithms and/or instructions. In embodiments, the one or more processors 110, 122 may include a desktop computer, mainframe computer system, workstation, image computer, parallel processor, or other computer system (e.g., networked computer) configured to execute a program configured to operate the system 100, as described throughout the present disclosure. It should be recognized that the steps described throughout the present disclosure may be carried out by a single computer system or, alternatively, multiple computer systems. In general, the term "processor" may be broadly defined to encompass any device having one or more processing elements, which execute program instructions from a non-transitory memory medium 112, 124. Moreover, different subsystems of the system 100 may include a processor or logic elements suitable for carrying out at least a portion of the steps described throughout the present disclosure.

The memory 112, 124 may include any memory medium known in the art suitable for storing program instructions executable by the associated one or more processors 110, 122. For example, the memory 112, 124 may include, but is not limited to, a read-only memory, a random-access memory, a magnetic or optical memory device (e.g., disk), a magnetic tape, a solid-state drive, and the like. In embodiments, the memory 112, 124 is configured to record power data and/or the output of the various data processing steps described herein. It is further noted that memory 112, 124 may be housed in a common controller housing with the one or more processors 110, 122. In an alternative embodiment, the memory 112, 124 may be located remotely with respect to the physical location of the processors 110, 122. For instance, the one or more processors 110, 122 may access a remote memory (e.g., server), accessible through a network (e.g., internet, intranet, and the like).

It is further noted that, while FIGS. 1A-1B depicts the sensor controller 120 as being embodied separately from the cooling system controller 108, such a configuration of system 100 is not a limitation on the scope of the present disclosure, but is provided merely for illustrative purposes. For example, the sensor controller 120 may be integrated within the cooling system controller 108.

The herein described subject matter sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components and/or wirelessly interactable and/or wirelessly interacting components and/or logically interacting and/or logically interactable components.

As used herein, the term "coupled" may generally refer to any type or configuration of coupling that is or becomes known or practicable. Coupling may be descriptive, for example, of two or more objects, devices, and/or components that are communicatively coupled, mechanically coupled, electrically coupled, and/or magnetically coupled. The term "communicatively coupled" generally refers to any type or configuration of coupling that places two or more objects, devices, components, or portions, elements, or combinations thereof in communication. Mechanical, electrical, and magnetic communications are examples of such communications. The term "mechanically coupled" generally refers to any physical binding, adherence, attachment, and/or other form of physical contact between two or more objects, devices, components, or portions, elements, or combinations thereof.

Those skilled in the art will recognize that it is common within the art to describe devices and/or processes in the fashion set forth herein, and thereafter use engineering practices to integrate such described devices and/or processes into data processing systems. That is, at least a portion of the devices and/or processes described herein can be integrated into a data processing system via a reasonable amount of experimentation. Those having skill in the art will recognize that a typical data processing system generally includes one or more of a system unit housing, a video display device, a memory such as volatile and non-volatile memory, processors such as microprocessors and digital signal processors, computational entities such as operating systems, drivers, graphical user interfaces, and applications programs, one or more interaction devices, such as a touch pad or screen, and/or control systems including feedback loops (e.g., feedback for sensing power; control circuitry for controlling power switching). A typical data processing system may be implemented utilizing any suitable commercially available components, such as those typically found in data computing/communication and/or network computing/communication systems.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. Furthermore, it is to be understood that the invention is defined by the appended claims.

The disclosure comprises the following items:
1. A system comprising:
   a sensor subsystem configured to be selectively powered by primary power and secondary power comprising:
   a sensor communicatively coupled to a cooling system controller and configured to detect a refrigerant;
   an alarm configured to receive an alarm signal when the refrigerant is detected by the sensor;
   a battery configured to power the sensor and the alarm with secondary power if primary power is disrupted; and
   a sensor controller communicatively coupled to the sensor, the alarm, and the cooling system controller, and configured to receive the primary power and distribute the primary power to the sensor and the alarm, the sensor controller configured to execute one or more steps comprising:
      at least one of detecting a disruption of the primary power or determining a planned disruption of the primary power; and
      upon a detection of the disruption of the primary power or determining a planned disruption of the planned disruption, of the primary power, causing the sensor controller to receive the secondary power from the battery.
2. The system of item 1, wherein the sensor controller comprises one or more processors, whereupon the disruption, or the planned disruption, of power to the cooling system controller, the one or more processors are configured to execute a set of program instructions stored in memory, wherein the set of program instructions includes at least one of the one or more steps.
3. The system of item 1 or item 2, wherein the set of program instructions is further configured to cause the one or more processors to:
   upon the disruption, or the planned disruption, of the primary power, cause the sensor and the alarm to receive the secondary power.
4. The system of any preceding item, wherein the battery is configured to charge via the primary power.
5. The system of any preceding item, wherein the refrigerant comprises an A2L refrigerant.
6. The system of any preceding item, wherein upon the sensor controller receiving secondary power from the battery, the sensor controller is further configured to:
   upon the sensor detecting the refrigerant, receiving a sensor signal from the sensor; and
   upon receiving the sensor signal, transmitting an alarm signal to the alarm.
7. The system of any preceding item, wherein the alarm is configured to emit at least one of an audible or a visual alarm upon receiving an alarm signal.
8. The system of any preceding item, wherein the alarm is configured to emit an audible and a visual alarm upon receiving an alarm signal.
9. The system of any preceding item, wherein the sensor is communicatively coupled to the sensor controller via a wireline connection via a ModBUS protocol.
10. A system comprising:
   a cooling system comprising:
      a refrigerant; and
      a cooling system controller; and
   a sensor subsystem comprising:
      a sensor communicatively coupled to the cooling system controller and configured to detect the refrigerant;
      an alarm configured to receive an alarm signal when the refrigerant is detected by the sensor;
      a battery configured to power the sensor and the alarm with secondary power if primary power is disrupted; and
      a sensor controller communicatively coupled to the sensor, the alarm, and the cooling system controller, and configured to receive primary power and distribute the primary power to the sensor and the alarm, the sensor controller comprising one or more processors, whereupon a disruption, or a planned disruption, of power to the cooling system, the one or more processors are configured to execute a set of program instructions stored in memory, the set of program instructions configured to cause the one or more processors to:
         at least one of detect the disruption of the primary power, or determine the planned disruption of the primary power; and
         upon a detection of the disruption of the primary power or a determination of a planned disruption of the primary power, cause the sensor controller to receive the secondary power from the battery.
11. The system of item 10, wherein the set of program instructions is further configured to cause the one or more processors to:
   upon the disruption, or the planned disruption, of the primary power, cause the sensor and the alarm to receive the secondary power.
12. The system of item 10 or item 11, wherein the battery is configured to charge via the primary power.
13. The system of any of items 10-12, wherein the refrigerant comprises an A2L refrigerant.
14. The system of any of items 10-13, wherein upon the sensor controller receiving secondary power from the battery, the sensor controller is further configured to:
   upon the sensor detecting the refrigerant, receiving a sensor signal from the sensor; and
   upon receiving the sensor signal, transmitting an alarm signal to the alarm.
15. The system of any of items 10-14, wherein the alarm is configured to emit at least one of an audible or a visual alarm upon receiving an alarm signal.
16. The system of any of items 10-15, wherein the alarm is configured to emit an audible and a visual alarm upon receiving an alarm signal.
17. A method of switching a power source for a refrigerant sensor in a cooling system comprising:
   operating a cooling system controller and a sensor controller with primary power;
   at least one of detecting a disruption of the primary power or determining a planned disruption of the primary power by the sensor controller; and
   upon the disruption, or the planned disruption, of the primary power, causing the sensor controller to receive secondary power from a battery.
18. The method of item 17, further comprising powering the refrigerant sensor with the secondary power from the sensor controller.
19. The method of item 17 or item 18, further comprising detecting a refrigerant by the refrigerant sensor, wherein the refrigerant comprises an A2L refrigerant.
20. The method of any of items 17-19, further comprising activating an alarm based on a detection of the A2L refrigerant by the refrigerant sensor.

## Claims

1. A system comprising:
a sensor subsystem configured to be selectively powered by primary power and secondary power comprising:
a sensor communicatively coupled to a cooling system controller and configured to detect a refrigerant;
an alarm configured to receive an alarm signal when the refrigerant is detected by the sensor;
a battery configured to power the sensor and the alarm with secondary power if primary power is disrupted; and
a sensor controller communicatively coupled to the sensor, the alarm, and the cooling system controller, and configured to receive the primary power and distribute the primary power to the sensor and the alarm, the sensor controller configured to execute one or more steps comprising:
at least one of detecting a disruption of the primary power or determining a planned disruption of the primary power; and
upon a detection of the disruption of the primary power or determining of the planned disruption of the primary power, causing the sensor controller to receive the secondary power from the battery.

2. The system of claim 1, wherein the sensor controller comprises one or more processors, wherein in response to the disruption, or the planned disruption, of power to the cooling system controller, the one or more processors are configured to execute a set of program instructions stored in memory, wherein the set of program instructions includes at least one of the one or more steps.

3. The system of claim 2, wherein the set of program instructions is further configured to cause the one or more processors to:
in response to the disruption, or the planned disruption, of the primary power, cause the sensor and the alarm to receive the secondary power.

4. The system of any preceding claim, wherein the battery is configured to charge via the primary power.

5. The system of any preceding claim, wherein the refrigerant comprises an A2L refrigerant.

6. The system of any preceding claim, wherein upon the sensor controller receiving secondary power from the battery, the sensor controller is further configured to:
in response to the sensor detecting the refrigerant, receive a sensor signal from the sensor; and
in response to receiving the sensor signal, transmit an alarm signal to the alarm.

7. The system of any preceding claim, wherein the alarm is configured to emit at least one of an audible or a visual alarm in response to receiving the alarm signal.

8. The system of claim 7, wherein the alarm is configured to emit an audible and a visual alarm in response to receiving the alarm signal.

9. The system of any preceding claim, wherein the sensor is communicatively coupled to the sensor controller via a wireline connection via a ModBUS protocol.

10. A system comprising:
a cooling system comprising:
a refrigerant; and
a cooling system controller; and
the system of any preceding claim.

11. A method of switching a power source for a refrigerant sensor in a cooling system comprising:
operating a cooling system controller and a sensor controller with primary power;
at least one of detecting a disruption of the primary power or determining a planned disruption of the primary power by the sensor controller; and
in response to the disruption, or the planned disruption, of the primary power, causing the sensor controller to receive secondary power from a battery.

12. The method of claim 11, further comprising powering the refrigerant sensor with the secondary power from the sensor controller.

13. The method of claim 11 or claim 12, further comprising detecting a refrigerant by the refrigerant sensor, wherein the refrigerant comprises an A2L refrigerant.

14. The method of claim 13, further comprising activating an alarm based on a detection of the A2L refrigerant by the refrigerant sensor.
